**Europäisches Patentamt**

**European Patent Office**

**Office européen des brevets**

(19)

(11) Veröffentlichungsnummer: **0 097 851**
**A1**

(12) ## EUROPÄISCHE PATENTANMELDUNG

(21) Anmeldenummer: **83105642.9**

(22) Anmeldetag: **09.06.83**

(51) Int. Cl.³: **A 61 B 10/00**

(30) Priorität: **24.06.82 DE 3223657**

(43) Veröffentlichungstag der Anmeldung:
**11.01.84 Patentblatt 84/2**

(84) Benannte Vertragsstaaten:
**CH FR GB IT LI SE**

(71) Anmelder: **Menke, Ernst, Dr. Dr. med.**
**Osterdeich 139**
**D-2800 Bremen 1(DE)**

(72) Erfinder: **Menke, Ernst, Dr. Dr. med.**
**Osterdeich 139**
**D-2800 Bremen 1(DE)**

(74) Vertreter: **Goddar, Heinz J., Dr. et al,**
**FORRESTER & BOEHMERT Widenmayerstrasse 4/1**
**D-8000 München 22(DE)**

(54) **Verfahren und Vorrichtung zum Erkennen des Zeitpunkts des Eisprungs bei Frauen.**

(57) Vorrichtung zum Erkennen des Zeitpunktes des Eisprungs im weiblichen Zyklus durch regelmäßiges Ermitteln der Basaltemperatur mit einem Temperaturfühler, wobei der Temperaturfühler auf der Unterseite einer Armbanduhr eingebracht ist, welche weiter eine Einrichtung zum Erfassen und Speichern der zu einem bestimmten Zeitpunkt alle 24 Stunden vorliegenden Temperatur der Frau sowie zum Vergleichen dieses Temperaturwertes mit dem 24 Stunden/ oder 48 Studen zuvor erfaßten und gespeicherten Temperaturwertes aufweist.

**EP 0 097 851 A1**

Croydon Printing Company Ltd.

Dr. Dr. med. Ernst Henke, Osterdeich 139, 2800 Bremen 1
------------------------------------------------------------

Verfahren und Vorrichtung zum Erkennen des Zeitpunkts
des Eisprungs bei Frauen

------------------------------------------------------------

Die Erfindung betrifft eine Vorrichtung zum Erkennen
des Zeitpunktes des Eisprungs im weiblichen Zyklus
durch regelmäßiges Ermitteln der Basaltemperatur mit
einem Temperaturfühler.

Von den 30 Tagen eines Monats ist eine Frau im gebährfähigen Alter nur drei bis vier Tage fruchtbar. Diese
kurze Fruchtbarkeitsperiode innerhalb eines Monats auf
einfache Weise erkennen zu können ist zum Zwecke der Verhinderung von ungewollten Schwangerschaften oder auch
bei Kinderwunsch im Rahmen der Familienplanung wichtig.
Es wird zu diesem Zwecke der Umstand genutzt, daß im
Laufe des weiblichen Sexualzyklus zu Beginn der fruchtbaren Tage die Körpertemperatur innerhalb weniger Stunden um 0,7 bis 0,8°C ansteigt, was auf die mit dem Eisprung verbundene Hormonumstellung zurückzuführen ist.

Es ist ein beschriebenes Verfahren, den Zeitpunkt des
Eisprungs durch tägliches Messen und Aufzeichnen der sogenannten "Aufwachtemperatur" festzustellen. Dieses Verfahren setzt voraus, daß die Frau jeden Morgen vor dem
Aufstehen ihre Köpertemperatur mißt und vermerkt. Die
ärztliche Erfahrung zeigt jedoch, daß die Aufzeichnungen
der Basaltemperaturen in vielen Fällen wenig exakt sind,
weil das Messen schlicht vergessen wird, oder aber das
Messen erst vorgenommen wird, nachdem die Frau bereits
aufgestanden ist, was zu einer Verfälschung der Aufwachtemperatur führt, weil der Ruhekörperwärme dann eine ungewisse motorisch bedingte Wärme hinzuaddiert ist, die
das Meßergebnis verfälscht.

Ein besonderes Thermometer zur Durchführung dieses Verfahrens ist aus der DE-OS 24 49 165 bekannt. Dieses
Thermometer hat jedoch den Nachteil, daß es von der Frau
unmittelbar nach dem Aufwachen in den Körper eingebracht
werden muß, weiter ist es hier erforderlich, die jeweils
gemessene Temperatur zu notieren.

Der Erfindung liegt daher die Aufgabe zugrunde, ein Verfahren und eine Vorrichtung zu schaffen, welche eine
sichere Feststellung des Anstiegs der Basaltemperatur

auch unabhängig von einem von der Frau in herkömmlicher Art und Weise bewußt vorgenommenen Meßvorgang mit einem Fieberthermometer zum Zeitpunkt des Aufwachens ermöglicht.

Erfindungsgemäß wird diese Aufgabe dadurch gelöst, daß der Temperaturfühler auf der Unterseite einer Armbanduhr eingebracht ist, welche weiter eine Einrichtung zum Erfassen und Speichern der zu einem bestimmten Zeitpunkt alle 24 Stunden vorliegenden Temperatur der Frau sowie zum Vergleichen dieses Temperaturwertes mit dem 24 Stunden und/oder 48 Stunden zuvor erfaßten und gespeicherten Temperaturwertes.

Die erfindungsgemäße Vorrichtung macht sich den Umstand zunutze, daß es zur Ermittlung des Anstieges der Basaltemperatur keineswegs darauf ankommt, diese im Zeitpunkt des Aufwachens der Frau zu ermitteln; vielmehr soll die Körpertemperatur ermittelt werden, die sich nach einigen Stunden Schlaf am frühen Morgen einstellt. Entsprechend wird zu einer vorgegebenen Uhrzeit - etwa 3 Uhr morgens oder 4 Uhr morgens - über den den Unterarm der Frau berührenden Temperaturfühler die Körpertemperatur der Frau gemessen und in einen in der Uhr befindlichen elektronischen Speicher eingeschrieben. Alle 24 Stunden wird zu exakt derselben Uhrzeit auf dieselbe Weise die Körpertemperatur der Frau ermittelt, dieser Wert wird mit dem und/oder den eingespeicherten Werten durch Bildung der Differenz der Beträge ermittelt. Übersteigt diese Differenz bzw. einer dieser Differenzen einen vorgegebenen Vergleichswert, der auf etwa 0,5°C eingestellt sein sollte, so indiziert dies einen kurz zuvor erfolgten Eisprung. Dieser Vorgang wiederholt sich alle 24 Stunden.

Es kann erforderlich sein, daß nicht nur der Differenzwert zu dem letzten (vor 24 Stunden ermittelten) Meßwert
gebildet wird, sondern auch zu dem vorletzten (vor 48
Stunden ermittelten) Meßwert, um ein exaktes Feststellen
des Eisprungs auch dann festzustellen, wenn der zu einem
bestimmten Zeitpunkt gemessene Temperaturwert gerade auf
der Anstiegsflanke des Anstiegs der Basaltemperatur liegt.

Vorteilhaft wird die gemessene Temperatur in dem Zahlenfeld einer Digital-Armbanduhr angezeigt, wobei das übliche Feld für die Darstellung der Uhrzeit verwendet
werden, aber auch ein besonderes Feld vorgesehen sein
kann. Wenn die Differenz zwischen dem zuletzt gemessenen
Temperaturwert und dem vor 24 Stunden (bzw. vor 48
Stunden) gemessenen Wert der Körpertemperatur den Vergleichswert übersteigt, so wird dies besonders angegeben,
beispielsweise durch eine blinkende Darstellung der gemessenen Temperatur und dadurch der vollzogene Eisprung
signalisiert.

Die erfindungsgemäße Vorrichtung ermöglicht also in
überraschend einfacher Weise die Basaltemperatur der Frau
zu einem exakt vorgegebenen Zeitpunkt regelmäßig zu erfassen und sogleich mittels Vergleichen dieses Temperaturwertes mit zuvor gemessenen Werten einen kurz zuvor
erfolgten Eisprung festzustellen.

Die in der vorstehenden Beschreibung sowie in den Ansprüchen offenbarten Merkmale der Erfindung können sowohl
einzeln als auch in beliebiger Kombination für die Verwirklichung der Erfindung in ihren verschiedenen Ausführungsformen wesentlich sein.

0097851

Ansprüche
=========

1. Vorrichtung zum Erkennen des Zeitpunktes des Eisprungs im weiblichen Zyklus durch regelmäßiges Ermitteln der Basaltemperatur mit einem Temperaturfühler,
dadurch gekennzeichnet, daß der Temperaturfühler auf
der Unterseite einer Armbanduhr eingebracht ist, welche
weiter eine Einrichtung zum Erfassen und Speichern der
zu einem bestimmten Zeitpunkt alle 24 Stunden vorliegenden Temperatur der Frau sowie zum Vergleichen dieses
Temperaturwertes mit dem 24 Stunden und/oder 48 Stunden
zuvor erfaßten und gespeicherten Temperaturwertes aufweist.

2. Vorrichtung zum Erkennen des Zeitpunktes des Eisprungs im weiblichen Zyklus nach Anspruch 1, dadurch
gekennzeichnet, daß die Einrichtung zu einem bestimmten
Zeitpunkt alle 24 Stunden den jeweils vorliegenden Temperaturwert erfaßt, mit dem vor 24 und/oder 48 Stunden erfaßten und gespeicherten Temperaturwert(en) vergleicht und bei Vorliegen einer einen vorgegebenen Vergleichswert überschreitenden Differenz zwischen dem vorliegenden und dem oder einem der vor 24 Stunden und/oder 48 Stunden zuvor erfaßten und gespeicherten Temperaturwert(en) eine besondere Anzeige bewirkt.

3. Vorrichtung nach Anspruch 1 oder 2, dadurch gekennzeichnet, daß der Temperaturfühler und die Einrichtung in einer Digital-Armbanduhr eingebracht sind.

4. Vorrichtung nach Anspruch 3, gekennzeichnet durch eine Anzeige der jeweils vorliegenden Temperatur im Ziffernfeld der Digital-Armbanduhr.

5. Vorrichtung nach Anspruch 4, gekennzeichnet durch eine blinkende Anzeige der jeweils vorliegenden Temperatur bei Vorliegen einer den vorgegebenen Vergleichswert überschreitenden Differenz.

Europäisches
Patentamt

**EUROPÄISCHER RECHERCHENBERICHT**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| X | US-A-4 151 831 (R.W. LESTER) <br> * Spalte 2, Zeile 21-27; Spalte 6, Zeilen 3-5; Abbildungen 8,9 * | 1-5 | A 61 B 10/00 |
| | --- | | |
| X | GB-A-2 066 528 (H. WOLFF et al.) <br> * Anspruch 7 * | 1,2 | |
| | --- | | |
| A | DE-A-2 803 152 (H. RÜCKSTÄDTER) | · | |
| | --- | | |
| X,P | DE-A-3 221 999 (G. BARNBECK) <br> * Ansprüche 1,2 * | 1,3 | |
| | ----- | | |

**RECHERCHIERTE SACHGEBIETE (Int. Cl. ³)**

A 61 B
G 01 K

Der vorliegende Recherchenbericht wurde für alle Patentansprüche erstellt.

| Recherchenort <br> DEN HAAG | Abschlußdatum der Recherche <br> 16-09-1983 | Prüfer <br> KNAUER F.E. |
|---|---|---|